(19) Europäisches Patentamt
European Patent Office
Office européen des brevets

(11) Veröffentlichungsnummer: **0 158 073**
**B1**

(12) # EUROPÄISCHE PATENTSCHRIFT

(45) Veröffentlichungstag der Patentschrift: **22.04.87**

(51) Int. Cl.⁴: **C 07 C 143/60**

(21) Anmeldenummer: **85102040.4**

(22) Anmeldetag: **25.02.85**

(54) **Verfahren zur Herstellung von Aminomethylen-2-aminonaphthalin-1-sulfonsäuren.**

(30) Priorität: **07.03.84 DE 3408300**

(43) Veröffentlichungstag der Anmeldung:
**16.10.85 Patentblatt 85/42**

(45) Bekanntmachung des Hinweises auf die Patenterteilung:
**22.04.87 Patentblatt 87/17**

(84) Benannte Vertragsstaaten:
**CH DE FR GB LI**

(56) Entgegenhaltungen:
**DE - A - 2 264 698**
**GB - A - 2 018 749**

(73) Patentinhaber: **BAYER AG, Konzernverwaltung RP Patentabteilung, D-5090 Leverkusen 1 Bayerwerk (DE)**

(72) Erfinder: **Stöhr, Frank-Michael, Dr., Weidenweg 25, D-5093 Burscheid (DE)**
Erfinder: **Schündehütte, Karl Heinz, Dr., Klief 75, D-5090 Leverkusen 3 (DE)**

EP 0 158 073 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Aminomethylen-2-aminonaphthalin-1-sulfonsäuren sind seit langem bekannte Zwischenprodukte für die Herstellung von Azofarbstoffen.

Ihre Herstellung erfolgt durch Kondensationsreaktionen der Aminonaphthalinsulfonsäuren bzw. der Acylaminonaphthalinsulfonsäuren mit N-Methylolamiden oder N-Methylolimiden wie N-Methylolbenzamid oder N-Methylolphthalimid in saurem Medium nach Tscherniak-Einhorn und anschliessender Verseifung (DE-OS 2 264 698).

Dieses Verfahren hat jedoch den Nachteil, dass man pro Mol Aminonaphthalinsulfonsäure 1 Mol N-Methylolamid bzw. -imid benötigt und demzufolge nach der Verseifung auch 1 Mol der entsprechenden Carbonsäure bzw. Dicarbonsäure freisetzt.

Gegenstand der vorliegenden Erfindung ist ein verbessertes Verfahren zur Herstellung von Verbindungen,
die in Form der freien Säure der Formel

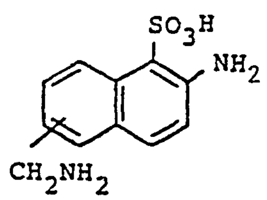

(I)

entsprechen, insbesondere solchen der Formel

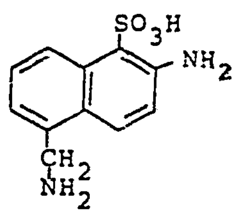

(II),

das dadurch gekennzeichnet ist, dass man Aminonaphthalinsulfonsäuren bzw. Acylaminonaphthalinsulfonsäuren, die in der Säureform der Formel

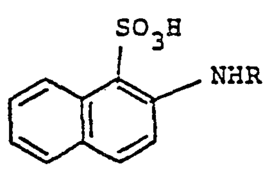

(III),

entsprechen, worin
R = H oder Acyl ist, wobei Acyl insbesondere für den Rest einer Carbonsäure oder einer Sulfonsäure vorzugsweise für $C_1$-$C_4$-Alkylcarbonyl- oder $C_1$-$C_4$-Alkyl-sulfonyl sowie gegebenenfalls substituiertes Phenylcarbonyl oder Phenylsulfonyl vorzugsweise $-COCH_3$ steht, in saurem Medium mit Verbindungen der Formel

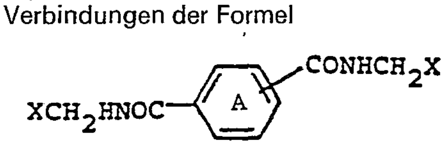

(IV),

worin
X für OH oder den rest einer anorganischen oder organischen Säure steht,
und der Ring A weitere übliche Substituenten, insbesondere OH, Halogen, oder Alkoxy aufweisen kann in saurem Medium, insbesondere bei pH-Werten unterhalb von 2, vorzugsweise in konz. Schwefelsäure, kondensiert, und anschliessend verseift.

Die Verseifung erfolgt bevorzugt in wässerigalkalischem Medium, wie beispielsweise in der DE-OS 2 264 698 beschrieben.

Die Umsetzung mit den elektrophilen Reagentien IV erfolgt in bekannter Weise (vgl. z.B. Org. Reactions, Bd. 14, 1965, S. 124, Verlag John Wiley & Sons, New York-London-Sidney).

Der Vorteil des neuen Verfahrens besteht darin, dass man pro Mol Aminonaphthalinsulfonsäure nur ein halbes Mol der Verbindung IV benötigt, da bei der Kondensation völlig überraschend beide funktionellen gruppen abreagieren und zu einem einheitlichen Produkt führen. Nach der Verseifung muss man demzufolge auch nur ein halbes Mol Benzoldicarbonsäure entsorgen. Ein weiterer Vorteil besteht darin, dass die Kondensation mit den Verbindungen der Formel IV überraschenderweise schneller verläuft als mit den bislang eingesetzten Verbindungen.

Von den Verbindungen der Formel IV sind die Verbindungen der Formel

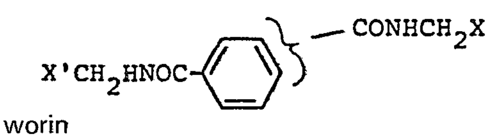

(IVa)

worin
X' OH, Halogen insbesondere Chlor oder Brom oder −OAc bedeutet, bevorzugt wobei −OAc insbesondere für $C_1$-$C_4$-Alkylcarbonyloxy vorzugsweise $-OCOCH_3$ steht,
ganz besonders bevorzugt ist die Verbindung der Formel

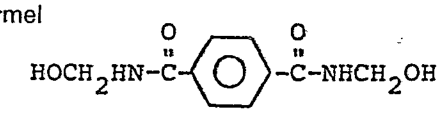

(IVb).

*Beispiel :*

60 g N,N'-Dimethylolterephthalsäurediamid werden in 730 g konz. Schwefelsäure bei 7-13° C eingetragen. Anschliessend trägt man 109 g 2-Amino-1-naphthalinsulfonsäure (Tobiassäure) bei 5-10° C ein und rührt 12 Stunden bei Raumtemperatur. Dann trägt man die klare, dunkle Lösung auf 1500 g Eis aus. Der Niederschlag wird abgesaugt und mit Wasser zur Entfernung der anhaftenden Schwefelsäure gewaschen. Die Paste wird in 800 ml Wasser gerührt und mit konz. Natronlauge neutralisiert. Nach Erreichen des Neutralpunktes setzt man noch soviel konz. NaOH zu, dass eine ca. 2n-NaOH entsteht. Zur Verseifung des entstandenen Terephthalsäurebisamids wird im Autoklaven 4 Stunden auf 140° C erwärmt. Nach dem Abkühlen auf Raumtemperatur erhält man 1600 ml einer klaren Lösung der 5-Aminomethylentobiassäure (95,5% Ausbeute durch Diazotieren). Zur Ausscheidung des Reaktionsproduktes wird der pH auf 7,5 gestellt. Die 5-Aminomethylentobiassäure fällt als schwerlösliches inneres Salz aus. Nach dem Isolieren erhält man 130,5 g einer farblosen Paste. (Die Signale des NMR-Spektrums der hergestellten Verbindung sind identisch mit den in der DE-OS 2 264 698 angegebenen Signalen (Beispiel 1).

### Patentansprüche

1. Verfahren zur Herstellung von Naphthalinderivaten der Formel

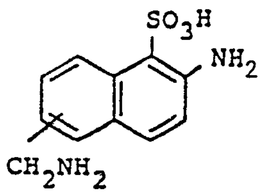

dadurch gekennzeichnet, dass man Naphthalinderivate der Formel

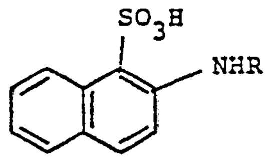

worin R = H oder Acyl in saurem Medium mit Verbindungen der Formel

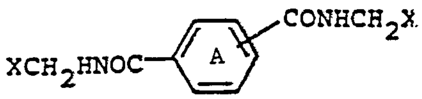

kondensiert,
worin X = OH oder Rest einer anorganischen oder organischen Säure und worin der Ring A weiter substituiert sein kann und anschliessend verseift.

2. Verfahren nach Anspruch 1, dadurch gekennzeichnet, dass man mit der Verbindung der Formel

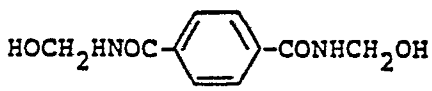

kondensiert.

3. Verfahren nach Ansprüchen 1 und 2, dadurch gekennzeichnet, dass man die Kondensation in konzentrierter Schwefelsäure durchführt.

### Claims

1. Process for the preparation of naphthalene derivatives of the formula

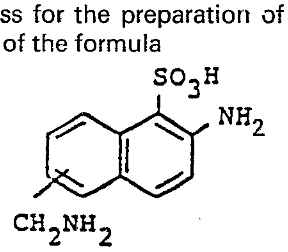

characterised in that naphthalene derivatives of the formula

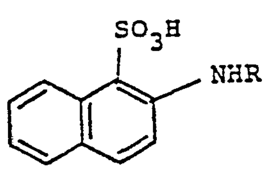

wherein
R = H or acyl, are subjected to a condensation reaction with compounds of the formula

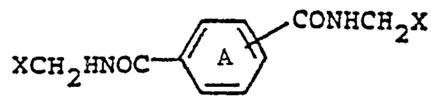

wherein
X = OH or the radical of an inorganic or organic acid, and wherein the ring A can be further substituted, in an acid medium and the product is then hydrolysed.

2. Process according to Claim 1, characterised in that the condensation reaction is carried out with the compound of the formula

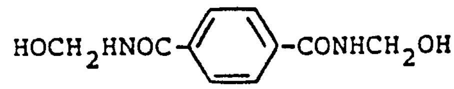

3. Process according to Claims 1 and 2, characterised in that the condensation reaction is carried out in concentrated sulphuric acid.

### Revendications

1. Procédé de production de dérivés de naphtalène de formule

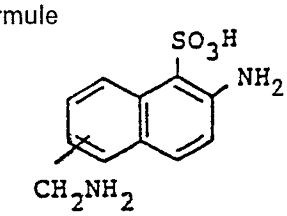

caractérisé en ce qu'on condense des dérivés de naphtalène de formule

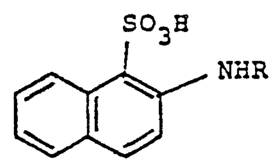

dans laquelle R représente H ou un groupe acyle en milieu acide avec les composés de formule

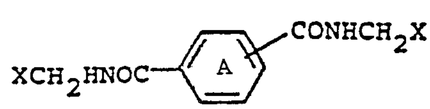

où X représente OH ou le reste d'un acide inorganique ou organique et le noyau A peut encore être substitué, puis on les saponifie.

2. Procédé suivant la revendication 1, caractérisé en ce qu'on effectue la condensation avec le composé de formule

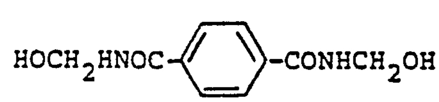

3. Procédé suivant les revendications 1 et 2, caractérisé en ce qu'on effectue la condensation dans de l'acide sulfurique concentré.